(19)

Europäisches Patentamt
European Patent Office
Office européen des brevets

(11) Veröffentlichungsnummer: **0 567 827 A1**

(12) **EUROPÄISCHE PATENTANMELDUNG**

(21) Anmeldenummer: **93105923.2**

(22) Anmeldetag: **13.04.93**

(51) Int. Cl.5: **C07D 231/12**, C07D 231/16, C07D 233/54, C07D 249/08

(30) Priorität: **30.04.92 DE 4214174**

(43) Veröffentlichungstag der Anmeldung: **03.11.93 Patentblatt 93/44**

(84) Benannte Vertragsstaaten: **BE DE FR GB NL**

(71) Anmelder: **BASF Aktiengesellschaft Carl-Bosch-Strasse 38 D-67063 Ludwigshafen(DE)**

(72) Erfinder: **Horchler von Locquenghien, Klaus, Dr. Rehbachstrasse 40 W-6708 Neuhofen(DE)**
Erfinder: **Hoelderich, Wolfgang, Prof. Dr. Mannheimer Strasse 18c W-6710 Frankenthal(DE)**
Erfinder: **Baus, Ulf, Dr. Im Hallgarten 6b W-6915 Dossenheim(DE)**
Erfinder: **Reuther, Wolfgang, Dr. Am Pferchelhang 16 W-6900 Heidelberg(DE)**
Erfinder: **Hahn, Erwin, Dr. Am Buechsenackerhang 31 W-6900 Heidelberg(DE)**

(54) **Verfahren zur Herstellung von N-Hydroxyazolen.**

(57) Verfahren zur Herstellung von N-Hydroxyazolen der allgemeinen Formel I

$$\overbrace{\underbrace{A\qquad N\text{-}OH}} \qquad (I),$$

in der
A für

$$-\overset{R^1}{\overset{|}{C}}=\overset{R^2}{\overset{|}{C}}-\overset{R^3}{\overset{|}{C}}=N- \quad , \quad -\overset{R^1}{\overset{|}{C}}=\overset{R^2}{\overset{|}{C}}-N=\overset{R^3}{\overset{|}{C}}- \quad ,$$

und

$$-\overset{R^1}{\overset{|}{C}}=N-\overset{R^2}{\overset{|}{C}}=N-$$

steht,
$R^1$,$R^2$,$R^3$ Wasserstoff, $C_1$- bis $C_4$-Alkyl und Halogen bedeuten,

EP 0 567 827 A1

indem man Azole der allgemeinen Formel II

A
N-H
(II),

in der A, $R^1$, $R^2$ und $R^3$ die oben genannten Bedeutungen haben, mit Wasserstoffperoxid oder Alkylperoxiden in Gegenwart von Zeolithkatalysatoren bei Temperaturen von (-20) bis 150 °C und Drücken von 0,1 bis 150 bar umsetzt.

Die vorliegende Erfindung betrifft ein Verfahren zur Herstellung von N-Hydroxyazolen wie N-Hydroxypyrazolen, N-Hydroxyimidazolen und N-Hydroxy-1,2,4-triazolen aus den entsprechenden Azolen mit Wasserstoffperoxid in Gegenwart von Zeolithen.

Die Oxidation von Pyrazol zu N-Hydroxypyrazol in homogener Phase unter basischen Reaktionsbedingungen mit Peroxocarbonsäuren ist aus der DE-A-38 20 739 und mit Diacylperoxiden aus der DE-A-38 20 738 bekannt.

Aus der EP-A-420 092 ist ein ähnliches Verfahren zur Darstellung von N-Hydroxy-1,2,4-triazol bekannt.

Die Nachteile bei diesen Verfahrensweisen liegen in der homogenen Reaktionsführung sowie in der Koppelproduktion von Salzen und Carbonsäuren in größeren Mengen als das jeweilige Wertprodukt. Weiterhin werden durch die verwendeten Oxidationsmittel erhöhte Anforderungen an das Reaktormaterial gestellt (Korrosion). Auch ist mit den typischen Problemen der Homogenkatalyse wie Katalysatorabtrennung, Katalysatorregenerierung und Katalysatoraufarbeitung zu rechnen.

Der vorliegenden Erfindung lag daher die Aufgabe zugrunde, den zuvor genanten Nachteilen abzuhelfen.

Demgemäß wurde ein neues und verbessertes Verfahren zur Herstellung von N-Hydroxyazolen der allgemeinen Formel I

$$\overset{\frown}{\underset{\smile}{A\quad}}\text{N-OH} \qquad \text{(I)},$$

in der
A für

$$-\overset{R^1}{\overset{|}{C}}=\overset{R^2}{\overset{|}{C}}-\overset{R^3}{\overset{|}{C}}=N- \quad , \quad -\overset{R^1}{\overset{|}{C}}=\overset{R^2}{\overset{|}{C}}-N=\overset{R^3}{\overset{|}{C}}- \quad ,$$

und

$$-\overset{R^1}{\overset{|}{C}}=N-\overset{R^2}{\overset{|}{C}}=N-$$

steht,
$R^1$,$R^2$,$R^3$ Wasserstoff, $C_1$- bis $C_4$-Alkyl und Halogen bedeuten,
gefunden, welches dadurch gekennzeichnet ist, daß man Azole der allgemeinen Formel II

$$\overset{\frown}{\underset{\smile}{A\quad}}\text{N-H} \qquad \text{(II)},$$

in der A, $R^1$, $R^2$ und $R^3$ die oben genannten Bedeutungen haben, mit Wasserstoffperoxid oder Alkylperoxiden in Gegenwart von Zeolithkatalysatoren bei Temperaturen von (-20) bis 150 °C und Drücken von 0,1 bis 150 bar umsetzt.

Das erfindungsgemäße Verfahren läßt sich wie folgt durchführen:
Das Azol II kann in einem Lösungsmittel gelöst mit dem Zeolith-Katalysator versetzt werden. Danach kann die Reaktionstemperatur und der Reaktionsdruck eingestellt werden. Anschließend wird Wasserstoffperoxid oder das Alkylperoxid zugegeben, vorzugsweise zugetropft.

Die Reaktionstemperatur liegt zwischen (- 20) und 150 °C, bevorzugt zwischen 0 und 120 °C, besonders zwischen 20 und 80 °C, der Druck beträgt 0,1 bis 150 bar, bevorzugt 0,5 bis 80 bar, besonders 0,8 bis 30

bar.

Das Verfahren kann diskontinuierlich in der Flüssigphase in Rührapparaturen durchgeführt werden, läßt sich aber auch in einem Rieselreaktor oder Sumpfphasenreaktor kontinuierlich durchführen.

Als Lösungsmittel können neben Wasser auch organische Lösungsmittel wie z.B. aliphatische Alkohole wie $C_1$- bis $C_4$-Alkanole, z.B. Methanol, Ethanol, n-Propanol, iso-Propanol, n-Butanol, iso-Butanol, sec.-Butanol und tert.-Butanol, Ketone wie $C_2$- bis $C_8$-Ketone z.B. Aceton und Methyl-ethylketon oder andere, mit Wasser mischbare Substanzen eingesetzt werden. Die besten Ergebnisse haben sich bei Verwendung von Methanol, Aceton, iso-Propanol oder tert.-Butanol sowie deren Gemischen ergeben.

Die eingesetzte Katalysatormenge zum Azol II liegt zwischen 0,1 und 200 Gew.-%, bevorzugt zwischen 5 und 100 Gew.-%, insbesondere zwischen 10 und 80 Gew.-%. Nach Beendigung der Reaktion wird der Katalysator abgetrennt, z.B. durch Filtration. Er kann anschließend für weitere Umsetzungen erneut eingesetzt werden. Die Aufarbeitung der Reaktionslösung erfolgt nach bekannten Methoden, z.B. wie in DE-A-38 20 739 beschrieben.

Wasserstoffperoxid oder ein Alkylperoxid dient als Oxidationsmittel für das erfindungsgemäße Verfahren. Wasserstoffperoxid kann vorzugsweise in Form seiner wäßrigen Lösungen eingesetzt werden. Man verwendet vorzugsweise 10 bis 50 Gew.-%ige wäßrige Lösungen, insbesonders 20 bis 35 Gew.-%ige wäßrige Wasserstoffperoxidlösungen. Ferner können auch Alkylperoxide eingesetzt werden wie $C_1$- bis $C_8$-Alkylhydroperoxide, z.B. tert.-Butylhydroperoxid und Cyclohexylhydroperoxid.

Das molare Verhältnis von Wasserstoffperoxid bzw. vom Alkylperoxid zum Azol II liegt in der Regel zwischen 0,5 : 1 und 5 : 1, bevorzugt zwischen 0,7 : 1 und 3 : 1 und besonders bevorzugt zwischen 0,9 : 1 und 1,5 : 1.

Die erfindungsgemäße Reaktion wird durch Zeolithe katalysiert.

Zeolithe sind bekanntermaßen kristalline Alumosilikate mit geordneten Kanal- und Käfigstrukturen, deren Porenöffnungen im Bereich von Mikroporen kleiner 1.2 nm liegen. Das Netzwerk solcher Zeolithe ist aufgebaut aus $SiO_4$- und $AlO_4$-Tetraedern, die über gemeinsame Sauerstoffbrücken verbunden sind.

Eine Übersicht der bekannten Strukturen findet sich beispielsweise bei W.M. Meier und D.H. Olson, "Atlas of Zeolite Structure Types", 2. Auflage, Butterworths, London 1987. Zum Ausgleich der negativen Elektrovalenz, die durch den Einbau von Al(III) in das Si(IV)-Silikatgitter entsteht, findet man bei Zeolithen austauschfähige Kationen, insbesondere kann es sich dabei je nach Herstellverfahren um Kationen des Natriums, Kaliums, Lithiums oder Cäsiums handeln. Ersetzt man diese Kationen gegen Protonen, beispielsweise durch einen Ionenaustausch, so erhält man die entsprechend aziden Festkörper mit Zeolithstruktur, die sogenannte H-Form.

Besonders vorteilhaft sind Zeolithe vom Pentasiltyp (MFI-Struktur; G.T. Kokotailo und W.M. Meier, Spec. Publ. Chem. Soc. 33 (1980) 133). Diese haben als Grundbaustein einen aus $SiO_4$-Tetraedern aufgebauten Fünfring gemeinsam. Sie sind durch ein hohes $SiO_2/Al_2O_3$-Ver-hältnis gekennzeichnet sowie durch Porengrößen, die zwischen denen der Zeolithe vom Typ A und denen vom Typ X oder Y liegen (vgl. Ullmanns Encyklopädie d. techn. Chem., a.a.O.).

Für das erfindungsgemäße Verfahren werden am besten titanhaltige Zeolithe eingesetzt. Aus US-A-3 329 481 sind Materialien bekannt, bei denen im Silikatgitter anstelle des Si(IV) Titan als Ti(IV) sein soll. Diese Titanzeolithe, insbesondere solche mit einer Kristallstruktur vom MFI-Typ (vgl. Meier, Olson, a.a.O), sowie Möglichkeiten zu ihrer Herstellung sind beschrieben beispielsweise in US-A-4 410 501, EP-A-311 983, US-A-4 666 692, DE-A-30 47 798 oder in BE-A-10 01 038. Titanhaltige Zeolithe mit anderen Strukturen kennt man aus EP-A-405 978. Außer Silizium und Titan können solche Materialien auch zusätzliche Elemente wie Aluminium (DE-A-31 41 238), Gallium (EP-A-266 825), Bor (US-A-4 666 692) oder geringe Mengen an Fluor enthalten (EP-A-292 363).

Titanzeolithe mit MFI-Sturktur sind dafür bekannt, daß sie über ein bestimmtes Muster bei der Bestimmung ihrer Röntgenbeugungsaufnahmen sowie zusätzlich über eine Gerüstschwingungsbande im IR-Bereich bei etwa 950 cm-1 identifiziert werden können (DE-A-30 47 798; Snamprogetti) und sich damit von Alkalititanaten oder kristallinen und amorphen $TiO_2$-Phasen unterscheiden.

Von Titanzeolithen mit MFI-Struktur ist bekannt, daß sie sich als Katalysatoren für Oxidationsreaktionen eignen (B. Notari, Stnd. Surf. Sci. Catal., Vol. 37, Amsterdam (1987), Seite 413 bis 425). So wird beispielsweise in EP-A-110 119 ein Verfahren beansprucht, wonach Propen in wäßriger Phase mit Wasserstoffperoxid an Titanzeolitehn zu Propylenoxid epoxidiert werden kann. Die Herstellung von Cyclohexanonoxim aus Cyclohexanon durch Umsetzung mit Ammoniak und Wasserstoffperoxid lehrt EP-A-208 311 und die Hydroxylierung von Aromaten kennt man aus GB-A-2 116 974. Die Oxidation gesättigter Kohlenwasserstoffe von C2-C18 mit $H_2O_2$ an o.g. Titanzeolithen beschreibt EP-A-376 453. Aus EP-A-314 147 ist bekannt, daß aliphatische sekundäre Amine mit diesen Katalysatoren zu den entsprechenden Hydroxylaminen oxidiert werden können. Diese Reaktion ist aus Houben-Weyl, Methoden der Chemie, 4. Auflage, Bd. E 16a, Seite

178-186 (1990), bereits bekannt und zeigt nicht die Probleme wie man sie bei der Hydroxylierung von Azolen findet.

Die nach den beschriebenen Verfahren hergestellten Titansilikatzeolithe können eingesetzt werden zur katalytischen Umwandlung organischer Moleküle. Umsetzungen dieser Art sind u.a. aus W. Hölderich, "Zeolites: Catalysts for the synthesis of organic compounds", Elsevier, Stud. Surf. Sci. Catal., Vol. 49, Amsterdam (1989), Seite 69 bis 93, und für mögliche Oxidationsreaktionen aus Stud. Surf. Sci. Catal., Vol. 37 (1987) 413 bis 425, bekannt.

Typischerweise stellt man die vorgenannten Titanzeolithe dadurch her, daß man eine wäßrige Mischung aus einer $SiO_2$-Quelle, einem Titandioxid und einer stickstoffhaltigen organischen Base, wie etwa Tetrapropylammoniumhydroxid, gegebenenfalls noch unter Hinzufügen von Alkali, in einem Druckbehälter unter erhöhter Temperatur im Zeitraum mehrerer Stunden oder weniger Tage umsetzt, wobei das kristalline Produkt entsteht. Dieses wird abfiltriert, gewaschen, getrocknet und zur Entfernung der organischen Stickstoffbase bei erhöhter Temperatur gebrannt. In dem so erhaltenen Pulver liegt das Titan zumindest teilweise innerhalb des Zeolithgerüsts in wechselnden Anteilen mit vier-, fünf- oder sechsfacher Koordination vor (J. Chem. Soc., Chem. Commun. 1991, S. 678 bis 680). Zur Verbesserung des katalytischen Verhaltens kann sich noch eine mehrmalige Waschbehandlung mit schwefelsaurer Wasserstoffperoxidlösung anschließend, worauf das Titanzeolith-Pulver erneut getrocknet und gebrannt werden muß, wie es etwa in EP-A-267 362 beschrieben wird. Der pulverförmige Titanzeolith kann mit einem geeigneten inerten Binder in einem Formgebungsschritt verarbeitet werden, um ihn als Katalysator in einer besser handhabbaren Form verfügbar zu machen. Eine Methode hierzu wird in EP-A-200 260 aufgezeigt.

In der DE-A-41 38 155 wird ein neues Verfahren zur Kristallisation von Titansilikaten mit Zeolithstruktur durch hydrothermale Umsetzung einer $SiO_2$-Quelle mit einer Titankomponente in Gegenwart wäßriger Lösungen von schwach konzentriertem Tetraalkylammoniumhalogeniden und zusätzlich Ammoniak beschrieben. Dieses Verfahren ermöglicht, daß man die entstehenden Titanzeolith-Kristalle in hoher Ausbeute direkt in Form plättchenförmiger, weitestgehend alkalifreier, großer Primärkristallite erhält, die aufgrund ihrer Teilchengröße ohne weitere Verformungsschritte als Katalysatoren zur Umsetzung organischer Moleküle und zwar insbesondere in Wirbelschicht-, Rieselbett- oder Suspensionsfahrweisen verwendet werden können.

Dabei kann man auf den oben beschriebenen Einsatz von teurem Tetrapropylammoniumhydroxid verzichten, wenn man stattdessen geringe Mengen billiger Tetrapropylammoniumhalogenide, beispielsweise Tetrapropylammoniumbromid (TPABr) verwendet und dabei ein molares Verhältnis von $TPABr/SiO_2$ von 0,042 : 1 bis 0,2 : 1, vorzugsweise aber von 0,042 : 1 bis 0,15 : 1 in der Reaktionsmischung einhält. Als vorteilhaft hat es sich gezeigt, daß man die Kristallisation der Titansilikate mit Zeolithstruktur hydrothermal bei Temperaturen von 100 bis 250 °C, besonders bei 120 bis 200 °C und insbesondere bei 150 bis 190 °C durchführt. Dabei ist es angebracht, daß man dabei ein molares Verhältnis von als wäßrige Lösung eingesetztem Ammoniak zu $SiO_2$ von 10 : 1 bis 1 : 1, besser von 6 : 1 bis 1,5 : 1 und besonders bevorzugt von 5 : 1 bis 3 : 1 einhält.

Nach diesem Verfahren erhält man katalytisch verwendbare Titansilikat Zeolithe dadurch, daß man die Titankomponente in Form einer löslichen, wäßrigen oder wässrig-alkoholischen Peroxotitanatverbindung in der vorgeschriebenen Weise bei der hydrothermalen Umsetzung der Reaktionsmischung zusetzt. Dies gelingt dadurch, daß man als molare Verhältnisse der Reaktionsmischung für $SiO_2/TiO_2$ im Bereich von 10 bis 1500 und insbesondere von 10 bis 250, bevorzugt aber von 10 bis 100, arbeitet und/oder dabei eine Verdünnung von $SiO_2/H_2O$ mit 0,07 bis 0,25, bevorzugt von 0,05 bis 0,04, beachtet.

Das alkalifreie Verfahren macht es möglich, daß darüber hinaus das Material nach einer Temperaturbehandlung bei 350 bis 600 °C, bevorzugt bei 400 bis 550 °C und insbesondere vorteilhafterweise bei 450 bis 500 °C, direkt und ohne zusätzlichen Ionenaustausch und insbesondere aufgrund der Kristallgröße von mehr als 1 µm und der plättchenförmigen Kristallform ohne weitere Formgebung in einer katalytisch wirksamen Form vorliegt und als Katalysator verwendet werden kann.

Das Zwischenglied A und die Substituenten $R^1$, $R^2$ und $R^3$ haben folgende Bedeutungen:

A

$$-\overset{R^1}{\overset{|}{C}}=\overset{R^2}{\overset{|}{C}}-\overset{R^3}{\overset{|}{C}}=N- \quad , \quad -\overset{R^1}{\overset{|}{C}}=\overset{R^2}{\overset{|}{C}}-N=\overset{R^3}{\overset{|}{C}}- \quad ,$$

und

$$-C(R^1)=N-C(R^2)=N- ,$$

$R^1$,$R^2$,$R^3$

- Wasserstoff,
- $C_1$- bis $C_4$-Alkyl wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,
- Halogen wie Fluor, Chlor, Brom und Jod, bevorzugt Fluor, Chlor und Brom, besonders bevorzugt Fluor und Chlor.

Als Edukte seien unter den Azolen II genannt: Pyrazol, Imidazol, 1,2,4-Triazol, 3-Chlorpyrazol, 3-Methylpyrazol, 4-Methyl-pyrazol und 3,5-Dimethylpyrazol.

Als N-Hydroxyazole I seien beispielhaft genannt: N-Hydroxypyrazol, N-Hydroxyimidazol, N-Hydroxy-1,2,4-Triazol, N-Hydroxy-3-chlorpyrazol, N-Hydroxy-3-methylpyrazol, N-Hydroxy-4-methylpyrazol und N-Hydroxy-3,5-dimethylpyrazol.

N-Hydroxyazole I sind wertvolle Ausgangsstoffe zur Synthese von Verbindungen mit breitem biologischem Wirkungsspektrum. So wird z.B. in EP-A-388 665 die Herstellung von Insektiziden auf der Basis von N-Hydroxypyrazol und N-Hydroxytriazol beschrieben. Im Agrarbereich einsetzbare Fungizide lassen ich ebenfalls unter Verwendung von N-Hydroxypyrazol (DE-A-39 05 948) bzw. N-Hydroxytriazol (DE-A-39 06 771 und EP-A-421 227) darstellen. Die Synthese von Nitrifikationsinhibitoren gemäß DE-A-34 09 317 erfolgt gleichfalls unter Einsatz von N-Hydroxypyrazol.

Beispiele

Herstellung der Katalysatoren

Katalysator A

In einem mit Rührer und Rückflußkühler versehenen Glaskolben werden 112,5 g deionisiertes Wasser auf 5 °C abgekühlt. Dazu tropft man innerhalb von 15 Minuten 34,7 g Tetraisopropylorthotitanat und 203,6 g Wasserstoffperoxidlösung (30 Gew.-%). Zu der entstandenen orangeroten Lösung fügt man 527,5 g einer Ammoniaklösung (25 Gew.-%) und läßt den entstandenen Ansatz sich über Nacht auf Raumtemperatur erwärmen. Abschließend wird für die Dauer von 3 Stunden auf 80 °C unter Rühren erwärmt. Eventuell eintretender Gewichtsverlust wird durch Zugabe einer entsprechenden Menge an Ammoniaklösung ausgeglichen. Die so hergestellte Lösung wird mit 73,5 g Tetrapropylammoniumbromid, 224,8 g Wasser und 264,1 g Ludox AS-40 Silicasol in einen Stahlautoklaven mit Rührvorrichtung eingefüllt.

Im Laufe von 168 Stunden bei einer Temperatur von 185 °C wird die Reaktionsmischung unter Rühren bei 100 U/min umgesetzt, nach dem Abkühlen das kristalline Produkt abfiltriert, neutralgewaschen, getrocknet und bei 500 °C innerhalb 5 Stunden in Luftatmosphäre kalziniert.

Das Produkt zeigt das typische Röntgendiffraktogramm des TS-1 Titansilikalits. Die Kristalle haben eine Größe von 2 bis zu 25 µm mit einem plättchenförmigem Habitus. Die IR-Aufnahme zeigt deutlich eine scharfe Bande bei 955 $cm^{-1}$. Die chemische Analyse ergibt ein molares Verhältnis im Produkt von Si/Ti = 16,6. Bezogen auf eingesetztes $SiO_2$ betrug die Ausbeute an kristallinem, kalzinierten Produkt 96,3 %.

Katalysator B

In einem mit Rührer und Rückflußkühler versehenen Glaskolben werden 45,2 g deionisiertes Wasser auf 5 °C abgekühlt. Dazu tropft man innerhalb von 15 Minuten 6,9 g Tetraisopropylorthotitanat und 81,5 g Wasserstoffperoxidlösung (30 Gew.-%). Zu der entstandenen orangeroten Lösung fügt man 211,0 g einer Ammoniaklösung (25 Gew.-%) und läßt den entstandenen Ansatz sich über Nacht auf Raumtemperatur erwärmen. Anschließend wird für die Dauer von 3 Stunden auf 80 °C unter Rühren erwärmt. Eventuell eintretender Gewichtsverlust wird durch Zugabe einer entsprechenden Menge an Ammoniaklösung ausgeglichen. Die so hergestellte Lösung wird mit 14,7 g Tetrapropylammoniumbromid, 75,5 g Wasser und 22,1 g Aerosil-200 (Degussa, pyrogene Kieselsäure) in einen Stahlautoklaven eingefüllt.

Im Laufe von 168 Stunden bei einer Temperatur von 185 °C wird die Reaktionsmischung umgesetzt, nach dem Abkühlen das kristalline Produkt abfiltriert, neutralgewaschen, getrocknet und bei 500 °C innerhalb 5 Stunden in Luftatmosphäre kalziniert.

Das Produkt zeigt das typische Röntgendiffraktorgramm des TS-1 Titansilikalits. Die Kristalle haben eine gleichförmige Größe von ca. 8 µm mit einem plättchenförmigen Habitus. Die IR-Aufnahme der Probe zeigt deutlich eine scharfe Bande bei 960 cm-1. Die chemische Analyse ergibt ein molares Verhältnis im Produkt von Si/Ti = 37,7. Die Verunreinigungen durch Alkali betrugen nur 0,0015 Gew.-% Natrium und 0,0045 Gew.-% an Kalium. Bezogen auf eingesetztes $SiO_2$ ergab sich eine Ausbeute an kristallinem, kalzinierten Produkt von 90,3 %.

Beispiele 1 bis 25

Die Reaktionen wurden in Glasautoklaven mit Magnetrührer (250 bis 1000 ml) in der Flüssigphase durchgeführt. Im einzelnen wurde dabei wie folgt vorgegangen: 10 g Azol wurden in 30 ml Isopropanol gelöst und mit der in den Tabellen 1 und 2 angegebenen Mengen Feststoffkatalysator versetzt. Dann wurde auf die angegebene Temperatur erhitzt und anschließend die entsprechende Menge einer wäßrigen Wasserstoffperoxidlösung zugetropft. Die Reaktionsmischung wurde so lange weiter gerührt bis kein Peroxid mehr nachweisbar war (Merck-Teststäbchen). Dann gab man weitere 30 ml Lösungsmittel auf einmal zu und filtrierte nach Abkühlung den Katalysator ab. Die Analyse der Reaktionslösung erfolgte in bekannter Weise durch geeichte HPLC. Umsätze und Selektivitäten finden sich ebenfalls in den Tabellen 1 und 2.

Tabelle 1

Hydroxylierung von Pyrazol

| Versuch Nr. | Katalysator | Verhältnis Azol/Kat. [g/g] | Verhältnis $H_2O_2$/Azol [mol/mol] | Temp. [°C] | Zeit [h] | Umsatz [Gew.-%] | Selekt. [mol-%] |
|---|---|---|---|---|---|---|---|
| 01 | A | 3,3 | 0,5 | 80 | 4,25 | 36,2 | 44,2 |
| 02 | A | 3,3 | 1,0 | 80 | 3,75 | 45,2 | 49,3 |
| 03 | A | 3,3 | 2,5 | 80 | 3,75 | 66,3 | 48,4 |
| 04 | B | 3,3 | 1,0 | 80 | 4,25 | 48,6 | 37,9 |
| 05 | B | 3,3 | 2,5 | 80 | 3,75 | 49,3 | 29,4 |
| 06 | A | 3,3 | 2,5 | 80 | 15,75 | 57,9 | 35,2 |
| 07 | A | 3,3 | 2,5 | 80 | 5,25 | 72,3 | 40,5 |
| 08 | A | 1,7 | 2,5 | 80 | 3,25 | 57,5 | 31,2 |
| 09 | A | 3,3 | 1,0 | 60 | 4,25 | 65,6 | 28,0 |
| 10 | B | 1,0 | 1,0 | 60 | 4,25 | 72,0 | 23,8 |
| 11 | B | 1,0 | 2,0 | 80 | 4,50 | 67,7 | 44,0 |
| 12 | B | 1,0 | 3,0 | 80 | 4,50 | 71,5 | 43,7 |
| 13 | A | 1,0 | 3,0 | 80 | 4,25 | 77,0 | 22,8 |
| 14 | B | 1,0 | 2,0 | 60 | 4,25 | 73,5 | 46,5 |
| 15 | A | 1,0 | 2,0 | 60 | 4,25 | 65,6 | 40,9 |
| 16 | A | 3,3 | 1,0 | 80 | 5,00 | 27,1 | 66,1 |

| Versuch Nr. | Katalysator | Verhältnis Azol/Kat. [g/g] | Verhältnis $H_2O_2$/Azol [mol/mol] | Temp. [°C] | Zeit [h] | Umsatz [Gew.-%] | Selekt. [mol-%] |
|---|---|---|---|---|---|---|---|
| 17 | B | 3,3 | 1,0 | 60 | 5,00 | 23,6 | 67,9 |
| 18 | A | 3,3 | 1,0 | 60 | 5,00 | 28,0 | 73,5 |
| 19 | A | 3,3 | 2,5 | 60 | 5,00 | 33,7 | 52,1 |

Tabelle 2

Hydroxylierung von Triazol

| Versuch Nr. | Katalysator | Verhältnis Azol/Kat. [g/g] | Verhältnis $H_2O_2$/Azol [mol/mol] | Temp. [°C] | Zeit [h] | Umsatz [Gew.-%] | Selekt. [mol-%] |
|---|---|---|---|---|---|---|---|
| 20 | A | 3,3 | 0,5 | 80 | 3,75 | 20,9 | 40,5 |
| 21 | B | 3,3 | 0,5 | 80 | 3,75 | 20,5 | 34,0 |
| 22 | A | 3,3 | 1,0 | 80 | 3,75 | 35,2 | 35,5 |
| 23 | B | 3,3 | 2,5 | 80 | 3,75 | 58,5 | 33,0 |
| 24 | A | 3,3 | 1,0 | 80 | 3,75 | 28,9 | 37,7 |
| 25 | A | 3,3 | 2,5 | 80 | 3,75 | 48,3 | 30,8 |

## Patentansprüche

**1.** Verfahren zur Herstellung von N-Hydroxyazolen der allgemeinen Formel I

(I),

in der
A für

und

$$-\overset{\overset{R^1}{|}}{C}=N-\overset{\overset{R^2}{|}}{C}=N-$$

steht,
$R^1,R^2,R^3$ Wasserstoff, $C_1$- bis $C_4$-Alkyl und Halogen bedeuten,
dadurch gekennzeichnet, daß man Azole der allgemeinen Formel II

A N-H (II),

in der A, $R^1$, $R^2$ und $R^3$ die oben genannten Bedeutungen haben, mit Wasserstoffperoxid oder Alkylperoxiden in Gegenwart von Zeolithkatalysatoren bei Temperaturen von (-20) bis 150 °C und Drücken von 0,1 bis 150 bar umsetzt.

**2.** Verfahren zur Herstellung von N-Hydroxyazolen I nach Anspruch 1, dadurch gekennzeichnet, daß man die Umsetzung an Zeolithkatalysatoren des Pentasil-Typs durchführt.

**3.** Verfahren zur Herstellung von N-Hydroxyazolen I nach Anspruch 1, dadurch gekennzeichnet, daß man als Zeolithkatalysatoren Titanzeolithe verwendet.

**4.** Verfahren zur Herstellung von N-Hydroxyazolen I nach Anspruch 1, dadurch gekennzeichnet, daß man die Umsetzung bei Temperaturen von 0 bis 120 °C durchführt.

**5.** Verfahren zur Herstellung von N-Hydroxyazolen I nach Anspruch 1, dadurch gekennzeichnet, daß man die Umsetzung bei Drücken von 0,5 bis 80 bar durchführt.

Europäisches Patentamt

# EUROPÄISCHER RECHERCHENBERICHT

Nummer der Anmeldung
EP 93 10 5923

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A<br>D | EP-A-0 347 689 (BASF AG)<br>* Anspruch 1 *<br>& DE-A-3 820 739 | 1 | C07D231/12<br>C07D231/16<br>C07D233/54<br>C07D249/08 |
| A<br>D | EP-A-0 347 676 (BASF AG)<br>* Anspruch 1 *<br>& DE-A-3 820 738 | 1 | |
| A,D | EP-A-0 420 092 (BASF AG)<br>* Anspruch 1 * | 1 | |
| A | EP-A-0 378 082 (BASF AG)<br>* Anspruch 4 * | 1 | |
| A,D | EP-A-0 385 224 (BASF AG)<br>* Seite 3, Zeile 28 - Zeile 48 * | 1 | |
| A,D | EP-A-0 314 147 (MONTEDIPE S.R.L.)<br>* Anspruch 1 * | 1,2 | |
| A,D | GB-A-2 116 974 (ANIC S.P.A.)<br>* Anspruch 1 * | 1,2 | **RECHERCHIERTE SACHGEBIETE (Int. Cl.5 )** |
| A,D | DE-A-3 047 798 (SNAM PROGETTI S.P.A.) | 2,3 | C07D |
| A,D | EP-A-0 311 983 (MONTEDIPE S.P.A.) | 2,3 | |
| A,D | EP-A-0 292 363 (RHONE-POULENC CHIMIE) | 2,3 | |
| A,D | US-A-3 329 481 (D.A. YOUNG) | 2,3 | |

Der vorliegende Recherchenbericht wurde für alle Patentansprüche erstellt

| Recherchenort | Abschlußdatum der Recherche | Prüfer |
|---|---|---|
| BERLIN | 05 AUGUST 1993 | HASS C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument